# EUROPEAN PATENT APPLICATION

(11) **EP 4 300 502 A1**
(43) Date of publication of application: **03.01.2024**
(21) Application number: 22182060.8
(22) Date of filing: 29.06.2022
(51) Int. Cl.: G16C 20/10

(54) **METHOD FOR DETERMINING THE COMPOSITION OF A REACTION SYSTEM**

(71) Applicant: Covestro Deutschland AG, 51373 Leverkusen (DE)
(72) Inventor: LOSCHEN, Christoph, 42781 Haan (DE); SCHNEIDER, Jakob, 50189 Elsdorf (DE); MASSOLLE, Anja, 48145 Münster (DE); MEYER, Jan, 42329 Wuppertal (DE)
(74) Representative: Levpat

(57) **Abstract**

The present invention pertains to a computer-implemented method which comprises the automated and rational (computer aided) modification of chemical recipes, in particular the design of the concrete starting composition of a reaction system, for example an oligomeric or polymeric polyol system, using an optimization procedure aiming at maximizing the similarity to an original target/reference system. Furthermore, the invention pertains to a data processing apparatus comprising means for carrying out the method, a computer program product comprising instructions which, when the program is executed by a computer, cause the computer to carry out the method and a computer-readable storage medium comprising instructions which, when executed by a computer, cause the computer to carry out the method.

## Description

The present invention relates to a computer-implemented method for determining the composition of a reaction system. The invention further relates to a data processing apparatus comprising means for carrying out the method, a computer program product comprising instructions which, when the program is executed by a computer, cause the computer to carry out the method and a computer-readable storage medium comprising instructions which, when executed by a computer, cause the computer to carry out the method.

The computer-aided modification of chemical information using an optimization procedure, in particular the design of a specific starting material composition for a reaction system, is the subject of ongoing efforts.

US 2020/342960 A1 discloses a target-based drug screening method using inverse quantitative structure-(drug)performance relationships (QSPR) analysis and molecular dynamics simulation. The method includes modeling a molecular structure of a test compound group against a target molecule, obtaining a quantitative structure-(drug)performance relationships (QSPR) of the test compound group, acquiring the optimal pharmacophore of a novel target-based drug through a numerical inversion of the QSPR, and selecting drug candidates having a molecular structure similar to the optimum pharmacophore from the test compound group. The approach is restricted to single molecules and not suited for reaction mixtures or polymers. The optimization is targeted directly towards experimental properties, that have either a high level of uncertainty when being predicted computationally or being scarcely available.

Maranas et al. address the design of polymers with optimal levels of macroscopic properties through the use of topological indices. Specifically, two zeroth-order and two first-order connectivity indices are for the first time employed as descriptors in structure-property correlations in an optimization study. Based on these descriptors, a set of new correlations for heat capacity, cohesive energy, glass transition temperature, refractive index, and dielectric constant are proposed. These correlations are incorporated into an optimization framework. (Camarda, Kyle V., and Costas D. Maranas. "Optimization in polymer design using connectivity indices." Industrial & Engineering Chemistry Research 38.5 (1999): 1884-1892.) This approach does not take into account the true polydisperse nature of polymers, i.e. is restricted to polymers modelled as infinite chains. The optimization is targeted directly towards experimental properties, that have either a high level of uncertainty when being predicted computationally or being scarcely available.

Faeder et al. describe rule-based modeling with BioNetGen to study the dynamics of complex biochemical systems. Rule-based models can be analyzed by carrying out deterministic or stochastic simulations. However, they are often difficult to calibrate due to many unknown parameters and limited experimental training data. They present a generic parameter estimation framework for calibrating rule-based models. The experimental data as well as qualitative properties of the system are encoded as a specification formula in a bounded linear temporal logic. Given a candidate set of parameter values they apply the statistical model checking procedure to evaluate the quality of this candidate set. Based on the outcome, a new set of parameters is chosen using a standard global search strategy. (Liu, Bing, and James R. Faeder. "Parameter estimation of rule-based models using statistical model checking." 2016 IEEE International Conference on Bioinformatics and Biomedicine (BIBM). IEEE, 2016.) This approach is limited to biological systems, i.e. not suited for polymer recipes. Furthermore is does not use an atomistic resolution of the species and hence is limited concerning the computation of detailed descriptors.

Daoutidis et al. have proposed a strategy that simultaneously identifies (a) the most desirable biomass-derived products for an application of interest and (b) the corresponding synthesis routes. The strategy consists of i) constructing an exhaustive network of reactions consistent with an input set of chemistry rules and ii) using the network information to formulate and solve an optimization problem that yields an optimal product distribution and the sequence of reactions that synthesize them. They use this strategy to identify potential renewable oxygenates and hydrocarbons obtained from heterogeneous catalysis of biomass that can be blended with gasoline to satisfy ASTM specifications. Multiple objectives (energy loss, catalyst requirement, and absolute heat duty) are considered, and multiple alternative solutions are found in each case (Marvin, W. Alex, Srinivas Rangarajan, and Prodromos Daoutidis. "Automated generation and optimal selection of biofuel-gasoline blends and their synthesis routes." Energy & Fuels 27.6 (2013): 3585-3594.) The approach is restricted to single molecules and not suited for realistic reaction mixtures or polymers. The optimization is targeted directly towards experimental properties, that have either a high level of uncertainty when being predicted computationally or being available only for small datasets.Mavrantzas et al. report a multi-scale model-based approach that combines a computer aided molecular design technique based on group contribution models for predicting polymer repeat unit properties with atomistic simulations for providing first-principles arrangements of the repeat units and for predictions of physical properties of the chosen candidate polymer structures. The method has been developed and tested for design of polymers with desired properties (Satyanarayana, Kavitha Chelakara, et al. "Computer aided polymer design using multi-scale modelling." Brazilian Journal of Chemical Engineering 27 (2010): 369-380.) The approach is restricted to polymers modelled as infinite chain of repeat units, not dedicated to realistic reaction mixtures or polydisperse polymers.

So far, no method has been described that optimizes a complex reaction system with respect to its composition using the similarity of a set of descriptors derived from its components at a monomeric and atomistic resolution.

Computer aided design methods in the art as mentioned above are usually based on computer-predicted properties that are used to define a desired target value or range for mostly well-defined single compounds or for polymers which are modelled as a monodisperse system of infinite chain length. In practice, those methods are hampered by several issues. First, the prediction of molecular properties with chemical accuracy for a molecule, a molecular mixtures or a polymer is still an unsolved challenge for many chemical systems and usually any property prediction is associated with a some inaccuracy, when compared to the experiment, that may deteriorate any subsequent optimization procedure. Secondly, the systems are always assumed to be of a monodisperse character, but for many systems, in particular oligomeric and polymeric ones, the polydisperse character is important, as for example different molecular chain lengths and/or the effect of end groups is non-negligible. In fact, most chemical systems are inherently mixtures with a complex composition instead of pure well-defined systems.

The present invention has the object of at least partially overcoming the drawbacks in the art. In particular, the invention has the object of improving the efficiency and accuracy of the creation of chemical reaction systems.

This object is achieved by a method according to claim 1. A data processing apparatus is the subject of claim 13, a computer program product the subject of claim 14 and a computer-readable storage medium the subject of claim 15. Preferred embodiments are the subject of the dependent claims. They may be combined freely unless the context clearly indicates otherwise.

Accordingly, a computer-implemented method for determining the composition of a reaction system comprises:
A) Providing information on a first reaction system, the information comprising the components of the first reaction system and their relative amounts in the first reaction system;
B) Providing information on a second reaction system, the information comprising the components of the second reaction system and their relative amounts in the second reaction system;
C) Providing at least one first rule for forming and/or cleaving chemical bonds from the components of the first reaction system and/or species formed from components of the first reaction system;
D) Applying the at least one rule on the first reaction system, thereby obtaining a modified first reaction system;
E) Providing at least one descriptor for at least part of the modified first reaction system and for at least part of a modified second reaction system as recited in step H);
F) Determining descriptor value(s) from the at least one descriptor for at least part of the modified first reaction system;
G) Providing at least one second rule for forming and/or cleaving chemical bonds from the components of the second reaction system and/or species formed from components of the second reaction system;
H) Applying the at least one second rule on the second reaction system, thereby obtaining a modified second reaction system;
I) Determining descriptor value(s) from the at least one descriptor as recited in step E) for at least part of the modified second reaction system;
J) Determining the distance and/or the dissimilarity between the values of the at least one descriptor from steps F) and I) using a measure for the distance in a descriptor space occupied by the descriptors;
K) Deciding to adapt or not to adapt the second reaction system;
L) If the decision to adapt in step K) has been taken, adapting the second reaction system by changing the components of and/or their relative amounts in the second reaction system yielding an updated second reaction system and subsequently repeating the method starting at step H) based on the updated second reaction system.

The method according to the example has several advantages. The method computes a descriptor-based similarity between a reference system and a new (2nd reaction system) system, it is not predicting just target properties for a new system. This avoids some problems due to inaccurately computed properties or insufficient models, which typically occur for complex chemical systems. In other words, not a reference range of properties is specified as objective for the optimization problem, but a reference reaction system, i.e. the first reaction system, which undergoes the same descriptor computation as the chemical system to be optimized, i.e. the second reaction system. The prediction of (target) properties is avoided at all and the overall optimization scheme is carried out using the concept of descriptor similarity. The underlying idea is to benefit from error cancellation, as the descriptors are computed for both systems involving the same approximations and assumptions in both cases.

Another advantage of the method is that it takes explicitly into account the polydisperse character of an oligomeric or polymeric reaction system, by using a kinetic model as basis for any subsequent computations, whereas so far only monodisperse pure and well-defined systems have been used. In addition, a complete recipe , i.e. a composition of a reaction mixture can be proposed with the proposed invention, whereas so far only pure and non-reactive single molecular structures are the targets of computer aided design.

Altogether, this approach allows for the rapid computer aided proposition of new chemical recipes of reactive systems that are most similar to a given reference system. This is particular useful for the efficient substitution of raw materials, e.g. by biobased materials, i.e. single or multiple ingredients of a chemical recipe, which have to be exchanged for example due to regulative or economic reasons, without compromising on the overall product's material properties. As the properties of the product created from the proposed recipe will already be very close to the reference system, much less experimental iterations are needed in order to adopt the recipe according to its changed ingredients.

According to the invention information on a first reaction system (step A)) and information on a second reaction system is provided (step B)). Both the first and the second reaction systems may possess a large variety of chemical components. According to the first embodiment of the invention the components of the first reaction system as recited in step A) and/or the components of the second reaction system as recited in step B) comprise alkylene oxides, polyfunctional carboxylic acids, carboxylic acid anhydrides, cyclic ethers, carbon dioxide, cyclic carbonates, polyols, polyisocyanates, polyamines, aromatic hydrocarbons, olefins, (meth)acrylates, bisphenols, phosgene, dialkyl carbonates, aldehydes, lactames, glycolides, amino acids, hydroxy-substituted carboxylic acids, or a combination of at least two of the aforementioned components.

According to step C) of the computer-implemented method according to the invention at least one first rule for forming and/or cleaving chemical bonds from the components of the first reaction system and/or species formed from components of the first reaction system is provided. Moreover, according to step G) at least one second rule for forming and/or cleaving chemical bonds from the components of the second reaction system and/or species formed from components of the second reaction system is provided.

According to an embodiment of the invention the at least one first rule as recited in C) comprises which functional group of a component is present, which functional group of a component is available for forming a covalent bond or for cleaving a covalent bond, which functional groups of components can react with other functional groups, a threshold criterion when the forming or the cleavage of a covalent bond occurs or a combination of at least two of the aforementioned rules.

Preferably the at least one first rule of step C) is identical to the at least one second rule of step G). This ensures that the first and second reaction system are modified according to similar chemical transformations.

According to step D) of the method according to the invention the at least one first rule is applied on the first reaction system, thereby obtaining a modified first reaction system. According to a preferred embodiment applying the at least one first rule as recited in step D) comprises running a kinetic Monte Carlo-simulation, running a molecular Monte Carlo-simulation, running a molecular dynamics simulation, running a Miller-Macosko type calculation. Here, especially a kinetic Monte-Carlo simulation proves to be very efficient and fast compared to other types of simulations.

Preferably the modified first reaction system obtained by applying the at least one first rule on the first reaction system, i.e. the reference system, comprises an ensemble of discrete components and information on the discrete components of the modified first reaction system and their relative amounts in the modified first reaction system.

According to step E) of the method according to the invention at least one descriptor for at least part of the modified first reaction system and for at least part of a modified second reaction system as recited in step H) is provided. In other words: according to the invention it is ensured that identical descriptors are used both for the modified first reaction system and for the modified second reaction system. This in turn ensures that a valid distance / similarity metric according to Step J) can be determined.

The at least one descriptor may be selected from a very large variety of descriptors. According to an embodiment of the invention the at least one descriptor as recited in step E) is a statistical/compositional descriptors, preferably number average molecular weight, weight average molecular weight, viscosity average molecular weight, the polydispersity index, the mean functionality, the OH number, the acid number.

It is also possible that the at least one descriptor is a topological descriptor, preferably Wiener Index, Randic connectivity index, Balaban-J index, Ipc index, Zagreb index, Bertz index, chi molecular connectivity indices, Kier-Hall valence connectivity index, seniority and priority indices, kappa shape indices, BCUT indices, Estate indices, Walk and path counts, extended connectivity fingerprints.

It is further possible that the at least one descriptor is a molecular descriptor, preferably Labute's approximate surface area, solvent-accessible surface area, radius of gyration, 2D autocorrelation, Eigenvalue-based descriptors, RDF descriptors, 3D-MoRSE descriptors, WHIM descriptors, GETAWAY descriptors, functional group counts, number of rotatable bonds, number of hydrogen acceptors and donors, Gasteiger/Marsili Partial Charges, topological polar surface area, molar refractivity.

It is further possible that the at least one descriptor is a force field based descriptors, preferably intramolecular potential energy, radial distribution function, bond angle distribution function, dihedral distribution functions; and/or a quantum chemical descriptor, preferably atomic charges, HOMO and LUMO energies, molecular hardness, molecular polarizability, dipole moment, total energy, molecular quantum number descriptor.

It is further possible that the at least one descriptor is a thermodynamic descriptor, preferably octanol-water partition coefficient, sigma-profile, sigma moment, polarity moment vector, activity coefficient, chemical potential, free energy, octanol-water partition coefficient, solubility;.

It is further possible that the at least one descriptor is a health, sustainability or environment related descriptor, preferably toxicity, carbon dioxide equivalents, carbon footprint, energy consumption, product recycling rate, sustainability index, supply chain miles, water footprint, price.

Preferably, a combination of at least two of the aforementioned descriptors is selected.

Using the at least one descriptor thus provided it is possible to determine one or more descriptor values from the at least one descriptor for at least part of the modified first reaction system as taught by step F) of the method according to the invention.

If the modified first reaction system comprises an ensemble of discrete components and information on the discrete components of the modified first reaction system and their relative amounts in the modified first reaction system, as mentioned above, it is preferred that the determination of the descriptor value(s) from the at least one descriptor for at least part of the modified first reaction system in step F) is carried out using atomistic resolution information for each discrete component. In case the at least one descriptor is a thermodynamic descriptor, as mentioned above, those descriptors preferably contain information concerning polarity and solubility, and are preferably obtained from the liquid phase thermodynamics approach COSMO-RS. Here, the previously obtained atomistic resolution information is used to construct the molecular input (σ-profiles) for the COSMO-RS calculation and descriptor generation

According to step G) of the method according to the invention at least one second rule for forming and/or cleaving chemical bonds from the components of the second reaction system and/or species formed from components of the second reaction system is provided.

According to a preferred embodiment of the invention the at least one second rule - as in case of the at least one first rule recited in step C) - comprises which functional group of a component is present, which functional group of a component is available for forming a covalent bond or for cleaving a covalent bond, which functional groups of components can react with other functional groups, a threshold criterion when the forming or the cleavage of a covalent bond occurs or a combination of at least two of the aforementioned rules.

As mentioned above it is possible that the at least one second rule may be identical to the at least one first rule.

According to step H) of the method according to the invention the at least one second rule is applied on the second reaction system, thereby obtaining a modified second reaction system. It is preferred that the at least one second rule again comprises running a kinetic Monte Carlo-simulation, running a molecular Monte Carlo-simulation, running a molecular dynamics simulation, running a Miller-Macosko type calculation.

Preferably the modified second reaction system obtained by applying the at least one second rule on the second reaction system, i.e. the system to be optimized - as in case of the modified first reaction system - comprises an ensemble of discrete components and information on the discrete components of the modified second reaction system and their relative amounts in the modified second reaction system.

In step I) of the method according to the invention one or more descriptor values are determined from the at least one descriptor as recited in step E) for at least part of the modified second reaction system.

Again, if the modified second reaction system comprises an ensemble of discrete components and information on the discrete components of the modified second reaction system and their relative amounts in the modified second reaction system, as mentioned above, it is preferred that the determination of the descriptor value(s) from the at least one descriptor for at least part of the modified second reaction system in step I) is carried out using atomistic resolution information for each discrete component.

With the descriptor value(s) for both the modified first reaction system and the modified second reaction system being determined according to steps F) and I) it is possible to determine (step J)) the distance and/or the dissimilarity between the values of the at least one descriptor from steps F) and I) using a measure for the distance in a descriptor space occupied by the descriptors.

In a preferred embodiment the distance and/or dissimilarity as recited in step J) comprises the euclidean/L2 distance, the manhattan/L1/cityblock distance, Canberra distance, Chebyshev distance, Mahalanobis distance, Minkowski distance, Rogers-Tanimoto dissimilarity, Russell-Rao dissimilarity, Sokal-Michener dissimilarity, Sokal-Sneath, mean absolute percentage error, Yule dissimilarity, cosine dissimilarity, dice dissimilarity or a combination of at least one of the aforementioned distances and/or dissimilarities.

Depending on the result of the determination of the distance and/or dissimilarity in step J) it is decided whether to adapt or not to adapt the second reaction system. Many different decision criteria may be applied. According to preferred embodiment the decision as recited in step K) comprises one or more of the following a decision criteria: a threshold value for the distance and/or dissimilarity in step J); a threshold value for the change in distance and/or dissimilarity relative to one or more previously executed steps J); a threshold value for the execution time for the method; a threshold value for the number of repetitions of the method; a threshold value for at least one of the descriptors. Here, the application of a threshold value for the change in distance and/or dissimilarity relative to one or more previously executed steps J) is preferred.

If the decision to adapt the second reaction system, i.e. the system to be optimized, in step K) has been taken the second reaction system is adapted in step L) of the method by changing the components of and/or their relative amounts in the second reaction system thus yielding an updated second reaction system.

Preferably, the adaptation of the second reaction system as recited in step L) is carried out by executing an algorithm selected from: random search, grid search, Bayesian optimization, simplex optimization, evolutionary optimization, genetic algorithm, particle swarm optimization, Metropolis-Hastings Markov-chain Monte-Carlo, adaptive Markov-chain Monte-Carlo, simulated annealing, parallel tempering, mixed linear and non-linear programming or a combination of at least two of the aforementioned algorithms.

Using the adapted second reaction system the method according to the invention is repeated starting at step H), i.e. by subsequently repeating the method starting at step H) based on the updated second reaction system. This means that the at least one second rule is applied also on the updated second reaction system, thereby again obtaining a modified (updated) second reaction system. Subsequently, the at least one descriptor as provided in step E) of the method according to the invention is used to again determine one or more descriptor values for at least part of the modified (updated) second reaction system.

This one or more descriptor value for the modified (updated) second reaction system is then used to again determine the distance and/or the dissimilarity between this/these value(s) and the value(s) obtained for the modified first reaction system in step F), i.e. the modified reference system.

If the adaption of the second reaction system in step L) has been carried out in a suitable way the distance/dissimilarity determined one more time in step J) should be reduced. This iteration of steps H) to L) with an updated second reaction system may be performed until the distance/dissimilarity drops below a value that is satisfactory for the user.

If, normally after a certain number of iterations, the value for the distance and/or the dissimilarity between the descriptor value(s) of the modified first reaction system and the descriptor value(s) drops below a certain threshold value it is decided in step K) to no longer adapt the second reaction system, i.e. the optimization of the second reaction system is completed. In this case an optimized or final second reaction system is proposed and preferably communicated to a user. It can be communicated by all suitable means to the user, e.g. by a personal computer or a mobile device.

Another aspect of the present invention relates to a data processing apparatus comprising means for carrying out the method of one of claims 1 to 12.

A further aspect of the present invention relates to a computer program product comprising instructions which, when the program is executed by a computer, cause the computer to carry out the method of one of claims 1 to 12.

An even further aspect of the present invention relates to a computer-readable storage medium comprising instructions which, when executed by a computer, cause the computer to carry out the method of one of claims 1 to 12.

The present invention will be further described with reference to the following figures without wishing to be limited by them

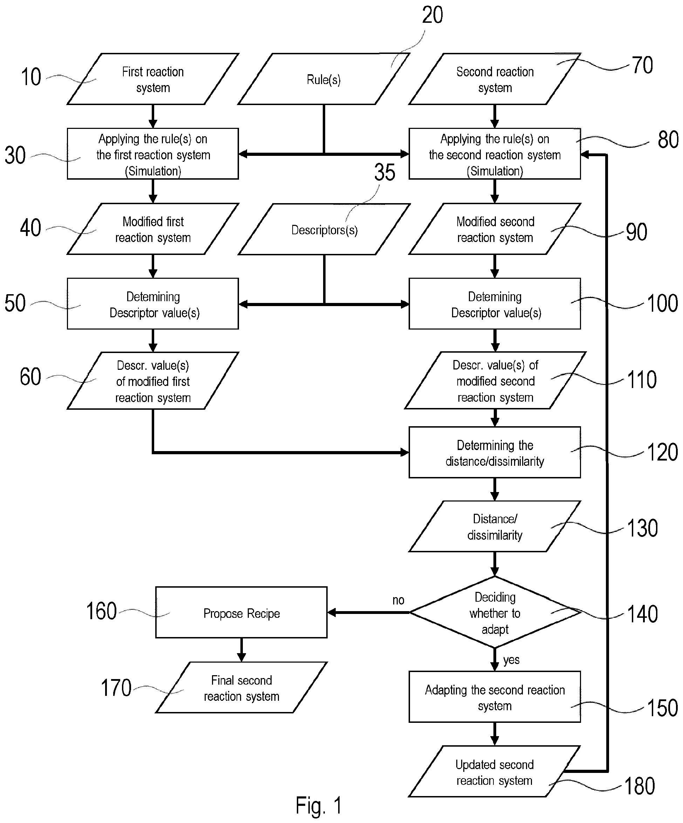

Fig. 1 shows a flow chart of a method according to the invention.

In connection with Fig. 1 an example workflow will be described in detail.

The example workflow is carried out as follows, with the aim to substitute starter molecules for a polyol recipe (the second reaction system) without compromising on its properties in a subsequent application e.g. in a polyurethane reaction. The workflow is fully automated and coded in Python.

A reference system 10 (the first reaction system) is defined to consists of the following chemical compounds, i.e. recipe: ethylene oxide (0.6), glycerin (0.2) and ethylene glycol (0.2) with the composition in mass fractions in parenthesis. Glycerin is defined to have three alcohol groups, ethylene glycol two alcohol groups and ethylene oxide one reactive epoxide group.

A kinetic Monte Carlo simulation (as for example in Suderman, Ryan, et al. "Generalizing Gillespie's direct method to enable network-free simulations." Bulletin of athematical biology 81.8 (2019): 2822-2848.) is set up where a number of kinetic rules 20 are defined to allow to react the alcohol groups with epoxide groups with a relative rate constant of 1, i.e. the functional groups of the identified components have the same reactivity. The kinetic rules 20 are applied on the first reaction system 10 (step 30) by carrying out the simulation using 100.000 initial seed molecules. A discrete representation of a polymer is obtained (the modified first reaction system 40), after all reactive groups have been reacted.

Subsequently a number of descriptors 35 is selected and corresponding descriptor values 60 are computed (step 50) from this modified first reaction system 40 as shown in Table 1: The selected descriptors 35 are: Polydispersity (PD), the OH number (OHZ), the average number of OH groups per molecule (fn(OH)), the topological polar surface area (TPSA) and the Wiener index as obtained from the molecular graph. Explanations regarding the Wiener Index can be found in Todeschini, Roberto, and Viviana Consonni. "Handbook of molecular descriptors", John Wiley & Sons, 2008. The descriptors values 60 are saved for later usage.

A new trial system (second reaction system 70) is then defined, using starters different from the first reaction system, i.e. the reference system, in the present case ethylene oxide, trimethylolpropane and 1,2-propylene glycol presently using the same set of kinetic rules 20 as defined for the first reaction system 10. This trial system is the system to be optimized in the present method. Trimethylolpropane is defined to have three alcohol groups, 1,2-propylene glycol two alcohol groups and ethylene oxide one reactive epoxide group. The composition in mass fractions is initiated with a random guess.

For the purpose of applying the kinetic rules 20 on the second reaction system 70 (step 80) a new kinetic Monte Carlo simulation is carried out and again a discrete representation of a polymer is obtained (the modified second reaction system 90) after all reactive groups have been reacted. The identical set of descriptors 35 is selected for the modified second reaction system 90 and the corresponding descriptor values 110 are computed (step 100, see Table 1).

The distance 130 in the descriptor space between the two modified reaction systems 40, 90 is then computed (step 120) by using the mean absolute percentage error between the descriptor vectors 60 of the modified reference system (modified first reaction system 40) and the descriptor vectors 110 of the trial system (modified second reaction system 90).

This distance 130 is then handed over to a Bayesian optimizer (as implemented in a Python library) including the actual composition of the second reaction system 70. Depending on the value of the distance 130 the Bayesian optimizer now decides to adapt the second reaction system 70 (step 140) or to not adapt the second reaction system 70 and propose a recipe (step 160). The decision criterion for the decision to be taken by the Bayesian optimizer is to stop after a maximum of 100 iterations, when no significant change to the distance metric has been recorded anymore. If the Bayesian optimizer decides to adapt the second reaction system 70 an updated second reaction system 180 is obtained by adaption of the second reaction system (step 150) for the next optimization step. The adaption may be effected by the optimizer suggesting new starters/mass fractions based on the optimization history.

The process 70-140 is iterated for approx. 100 iterations/cycles until no change in the distance 130 can be observed anymore or the change lies below a defined threshold . A final set of values of descriptors 110 is obtained with a minimum distance between the descriptors values 60, 110 of the reference/optimized system, i.e. the first and second modified reaction systems 40, 90:

**Table 1**

| System | PD | OHZ | Wiener Index | TPSA | fn(OH) |
|---|---|---|---|---|---|
| Reference recipe Modified first reaction system 40 | 1.18 | 727 | 11.6 | 71.9 | 2.4 |
| Proposed recipe Modified second reaction system 90 | 1.17 | 632 | 11.2 | 70.9 | 2.35 |

As a result the recipe of the second reaction system 70 is proposed (step 160), having the following chemical composition: ethylene oxide(0.54), trimethylolpropane(0.22) and 1,2-propylene glycol(0.24) which is the final second reaction system information 170. This proposed second reaction system 70 is then communicated to a user.

## Claims

1. A computer-implemented method for determining the composition of a reaction system, comprising:
A) Providing information on a first reaction system (10), the information comprising the components of the first reaction system (10) and their relative amounts in the first reaction system (10);
B) Providing information on a second reaction system (70), the information comprising the components of the second reaction system (70) and their relative amounts in the second reaction system (70);
C) Providing at least one first rule (20) for forming and/or cleaving chemical bonds from the components of the first reaction system (10) and/or species formed from components of the first reaction system (10);
D) Applying (30) the at least one first rule (20) on the first reaction system, thereby obtaining a modified first reaction system (40);
E) Providing at least one descriptor (35) for at least part of the modified first reaction system (40) and for at least part of a modified second reaction system as recited in step H);
F) Determining (50) descriptor value(s) (60) from the at least one descriptor (35) for at least part of the modified first reaction system (40);
G) Providing at least one second rule (20) for forming and/or cleaving chemical bonds from the components of the second reaction system (70) and/or species formed from components of the second reaction system (70);
H) Applying (80) the at least one second rule (20) on the second reaction system (70), thereby obtaining a modified second reaction system (90);
I) Determining (100) descriptor value(s) (110) from the at least one descriptor (35) as recited in step E) for at least part of the modified second reaction system (90);
J) Determining (120) the distance and/or the dissimilarity (130) between the values (60, 110) of the at least one descriptor (35) from steps F) and I) using a measure for the distance in a descriptor space occupied by the descriptors (35);
K) Deciding (140) to adapt or not to adapt the second reaction system (70);
L) If the decision to adapt in step K) has been taken, adapting (150) the second reaction system (70) by changing the components of and/or their relative amounts in the second reaction system (70) yielding an updated second reaction system (180) and subsequently repeating the method starting at step H) based on the updated second reaction system (180).

2. The method according to claim 1, wherein the components of the first reaction system (10) as recited in step A) and/or the components of the second reaction system (70) as recited in step B) comprise alkylene oxides, polyfunctional carboxylic acids, carboxylic acid anhydrides, cyclic ethers, carbon dioxide, cyclic carbonates, polyols, polyisocyanates, polyamines, aromatic hydrocarbons, olefins, (meth)acrylates, bisphenols, phosgene, dialkyl carbonates, aldehydes, lactames, glycolides, amino acids, hydroxy-substituted carboxylic acids, or a combination of at least two of the aforementioned components.

3. The method according claims 1 or 2, wherein the at least one first rule (20) recited in C) and/or the at least one second rule (20) recited in G) comprises which functional group of a component is present, which functional group of a component is available for forming a covalent bond or for cleaving a covalent bond, which functional groups of components can react with other functional groups, a threshold criterion when the forming or the cleavage of a covalent bond occurs or a combination of at least two of the aforementioned rules (20).

4. The method according to any one of the preceding claims, wherein the at least one first rule (20) of step C) is identical to the at least one second rule (20) of step G).

5. The method according to any one of the preceding claims, wherein applying (30) the at least one first rule (20) as recited in step D) and/or applying the at least one second rule (20) as recited in step H) comprises running a kinetic Monte Carlo-simulation, running a molecular Monte Carlo-simulation, running a molecular dynamics simulation, running a Miller-Macosko type calculation.

6. The method according to any one of the preceding claims, wherein the modified first reaction system (40) comprises an ensemble of discrete components and information on the discrete components of the modified first reaction system (40) and their relative amounts in the modified first reaction system (40), wherein determining (50) descriptor value(s) (60) from the at least one descriptor (35) for at least part of the modified first reaction system (40) in step F) is preferably carried out using atomistic resolution information for each discrete component.

7. The method according to any one of the preceding claims, wherein the modified second reaction system (90) comprises an ensemble of discrete components and information on the discrete components of the modified second reaction system (90) and their relative amounts in the modified second reaction system (90), wherein determining (100) descriptor value(s) (110) from the at least one descriptor (35) for at least part of the modified second reaction system (90) in step I) is preferably carried out using atomistic resolution information for each discrete component.

8. The method according to any one of the preceding claims, wherein the at least one descriptor (35) as recited in step E) is a statistical/compositional descriptors, preferably number average molecular weight, weight average molecular weight, viscosity average molecular weight, the polydispersity index, the mean functionality, the OH number; the acid number, and/or
a topological descriptor, preferably Wiener Index, Randic connectivity index, Balaban-J index, Ipc index, Zagreb index, Bertz index, chi molecular connectivity indices, Kier-Hall valence connectivity index, seniority and priority indices, kappa shape indices, BCUT indices, Estate indices, Walk and path counts, extended connectivity fingerprints; and/or
a molecular descriptor, preferably Labute's approximate surface area, solvent-accessible surface area, radius of gyration, 2D autocorrelations, Eigenvalue-based descriptors, RDF descriptors, 3D-MoRSE descriptors, WHIM descriptors, GETAWAY descriptors, functional group counts, number of rotatable bonds, number of hydrogen acceptors and donors, Gasteiger/Marsili Partial Charges, topological polar surface area, molar refractivity; and/or
a force field based descriptors, preferably intramolecular potential energy, radial distribution function, bond angle distribution function, dihedral distribution functions; and/or
a quantum chemical descriptor, preferably atomic charges, HOMO and LUMO energies, molecular hardness, molecular polarizability, dipole moment, total energy, molecular quantum number descriptor; and/or
a thermodynamic descriptor, preferably octanol-water partition coefficient, sigma-profile, sigma moment, polarity moment vector, activity coefficient, chemical potential, free energy, octanol-water partition coefficient, solubility; and/or
health, sustainability or environment related descriptors, preferably toxicity, carbon dioxide equivalents, carbon footprint, energy consumption, product recycling rate, sustainability index, supply chain miles, water footprint, price;
or a combination of at least two of the aforementioned descriptors.

9. The method according to any one of the preceding claims, wherein the distance and/or dissimilarity (130) as recited in step J) comprises the euclidean/L2 distance, the manhattan/L1/cityblock distance, Canberra distance, Chebyshev distance, Mahalanobis distance, Minkowski distance, Rogers-Tanimoto dissimilarity, Russell-Rao dissimilarity, Sokal-Michener dissimilarity, Sokal-Sneath, mean absolute percentage error, Yule dissimilarity, cosine dissimilarity, dice dissimilarity or a combination of at least one of the aforementioned distances and/or dissimilarities.

10. The method according to any one of the preceding claims, wherein the decision as recited in step K) comprises one or more of the following a decision criteria:
a threshold value for the distance and/or dissimilarity in step J),
a threshold value for the change in distance and/or dissimilarity relative to one or more previously executed steps J),
a threshold value for the execution time for the method,
a threshold value for the number of repetitions of the method,
a threshold value for at least one of the descriptors.

11. The method according to any one of the preceding claims, wherein adapting (150) the second reaction system (70) by changing the components of the second reaction system (70) and/or their relative amounts in the second reaction system (70) as recited in step L) comprises executing an algorithm selected from: random search, grid search, Bayesian optimization, simplex optimization, evolutionary optimization, genetic algorithm, particle swarm optimization, Metropolis-Hastings Markov-chain Monte-Carlo, adaptive Markov-chain Monte-Carlo, simulated annealing, parallel tempering, mixed linear and non-linear programming or a combination of at least two of the aforementioned algorithms.

12. The method according to claim 1, further comprising communicating a final second reaction system (170) to a user if the decision has been taken in step K) not to adapt the second reaction system in step K).

13. A data processing apparatus comprising means for carrying out the method of one of claims 1 to 12.

14. A computer program product comprising instructions which, when the program is executed by a computer, cause the computer to carry out the method of one of claims 1 to 12.

15. A computer-readable storage medium comprising instructions which, when executed by a computer, cause the computer to carry out the method of one of claims 1 to 12.
